(19) Europäisches Patentamt
European Patent Office
Office européen des brevets

(11) **EP 2 441 792 B1**

(12) **EUROPÄISCHE PATENTSCHRIFT**

(45) Veröffentlichungstag und Bekanntmachung des Hinweises auf die Patenterteilung:
**17.12.2014 Patentblatt 2014/51**

(21) Anmeldenummer: **11002082.3**

(22) Anmeldetag: **14.03.2011**

(51) Int Cl.:
*C08J 5/18* (2006.01)  *C08J 3/205* (2006.01)
*C08J 3/20* (2006.01)  *C08K 5/098* (2006.01)
*C08K 5/00* (2006.01)  *B01D 69/02* (2006.01)
*B01D 71/26* (2006.01)  *B29C 55/00* (2006.01)
*H01M 2/16* (2006.01)  *C07C 51/41* (2006.01)
*B01D 67/00* (2006.01)  *C07C 55/02* (2006.01)

(54) **Hochaktives beta-Nukleierungsadditiv für Polypropylen**

Highly active beta-nucleating agent for polypropylene

Additif de nucléation beta hautement actif pour polypropylene

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.10.2010 PCT/EP2010/006240**

(43) Veröffentlichungstag der Anmeldung:
**18.04.2012 Patentblatt 2012/16**

(73) Patentinhaber: **Treofan Germany GmbH & Co. KG 66539 Neunkirchen (DE)**

(72) Erfinder:
• **Busch, Detlef**
  **66740 Saarlouis (DE)**
• **Klein, Dominic**
  **66450 Bexbach (DE)**
• **Schmitz, Bertram**
  **57200 Sarreguemines (FR)**

(74) Vertreter: **Kremer, Viola**
  **Treofan Germany GmbH & Co. KG**
  **Am Prime Parc 17**
  **65479 Raunheim (DE)**

(56) Entgegenhaltungen:
**WO-A1-2011/047797    CN-A- 1 966 563**

• ZHAO S ET AL: "A highly active novel beta-nucleating agent for isotactic polypropylene", POLYMER, ELSEVIER SCIENCE PUBLISHERS B.V, GB, Bd. 49, Nr. 11, 27. Mai 2008 (2008-05-27), Seiten 2745-2754, XP022668139, ISSN: 0032-3861, DOI: 10.1016/J.POLYMER. 2008.04.012 [gefunden am 2008-04-12]

• MOHAMED M A ET AL: "A comparative study of the thermal reactivities of some transition metal oxalates in selected atmospheres", THERMOCHIMICA ACTA, ELSEVIER SCIENCE PUBLISHERS, AMSTERDAM, NL, Bd. 429, Nr. 1, 1. Mai 2005 (2005-05-01), Seiten 57-72, XP025386883, ISSN: 0040-6031 [gefunden am 2005-05-01]

• QIANG DOU: "Effect of Metallic Salts of Pimelic Acid and Crystallization Temperatures on the Formation of -beta- Crystalline Form in Isotactic Poly(propylene)", JOURNAL OF MACROMOLECULAR SCIENCE PART B. PHYSICS, MARCEL DEKKER INC., NEW YORK, NY, US, vol. 46, no. 6, 1 November 2007 (2007-11-01), pages 1063-1080, XP008148545, ISSN: 0022-2348, DOI: 10.1080/00222340701581334 [retrieved on 2007-11-06]

• ANONYMOUS: "BETA NUCLEATOR FOR POLYPROPYLENE", IP.COM JOURNAL, IP.COM INC., WEST HENRIETTA, NY, US, 2 September 2004 (2004-09-02), XP013021370, ISSN: 1533-0001

• QIANG DOU: "Effect of Metallic Salts of Glutaric Acid on the Formation of ?-crystalline Form in Isotactic Polypropylene", JOURNAL OF ELASTOMERS AND PLASTICS, SAGE PUBLICATIONS LTD, GB, vol. 41, 13 July 2009 (2009-07-13), pages 509-522, XP008160675, ISSN: 0095-2443, DOI: 10.1177/0095244309339789

**Beschreibung**

Die Erfindung betrifft ein Verfahren zur Erhöhung des Anteils der β-Kristallmodifikation in Polypropylen.

Von Polypropylen sind neben der amorphen Phase drei verschiedene kristalline, die α-, β-, und γ-Phasen bekannt. Beim Abkühlen von Polypropylenschmelzen bildet sich üblicherweise überwiegend das α-kristalline PP. Durch eine bestimmte Temperaturführung beim Abkühlen einer Polypropylenschmelze kann ein erhöhter Anteil an β-kristalliner Phase, erzeugt werden. Der auf diese Weise erzeugte Anteil an β-kristallinem PP beträgt weniger als 10%. Die hexagonale β-Modifikation des PP's zeichnet sich gegenüber der monoklinen α -Modifikation durch bessere mechanische Eigenschaften, insbesondere erhöhter Schlagzähigkeit und Spannungsrißbeständigkeit aus. Daneben weist die β-Modifikation des Polypropylens mit 148-155°C einen deutlich niedrigeren Schmelzpunkt gegenüber der α -Modifikation mit einem Schmelzpunkt von 160-170°C auf. Ein erhöhter Anteil an β -kristallinem Polypropylen wirkt sich daher in einigen Anwendungen günstig auf bestimmte Gebrauchseigenschaften des Polypropylens aus. Aus diesem Grund wurden in der Vergangenheit einige Additive entwickelt, die zu noch höheren Anteilen an Polypropylen in der β-Modifikation führen und daher im allgemeinen als β-Nukleatoren oder β -Nukleierungsmittel bezeichnet werden.

Als β-Nukleator mit hoher Aktivität ist der Farbstoff γ-Quinacridone in dem Deutschen Patent 1188278 beschrieben. Der Nachteil dieses Nukleierungsmittel ist jedoch die intensive Rotfärbung und die mangelnde thermische Stabilität, die oftmals beim Kompoundieren zur Zersetzung des Nukleierungsmittels und damit zum Verlust seiner Aktivität führt.

Im US-Patent 3540979 ist das Calciumsalz der Phtalsäure als thermisch stabiles β -Nukleierungsmittel beschrieben. Der Nachteil dieses Nukleierungsmittel ist die geringe Aktivität. Der damit erzielte Anteil an β-kristallinem Polypropylen beträgt höchsten 70% (K~0,5-0,7).

Ein zweikomponenten Nukleierungssystem aus Calciumcarbonat und organischen Dicarbonsäuren beschreibt DE 3610644. Dieses Nukleierungssystem zeigt in der Praxis jedoch eine schwankende Aktivität. Daher mangelt es an Reproduzierbarkeit. Der direkten Einsatz von Calciumsalze verschiedener Dicarbonsäuren ist im Patent DE 4420989 beschrieben. Die β-nukleierende Wirkung verschiedener Dicarboxamide, insbesondere N,N-Dicyclohexyl-2,6-Naphtalen-dicarboxamide beschreibt EP 0557721. Nachteil dieses Nukleators sind, die hohen Eduktkosten, sowie komplizierte Syntheseschritte bei der Herstellung.

Die Dokumente Dou, Q., J. Macromolecular Sc. Part B: Physics, Marcel Dekker Inc., New York, NY, US, Bd.46, Nr. 6, 1. Nov 2007 ()2007-11-01), S. 1063-1080, bzw. (Author unbekannt) IP.COM Journal, IP.COM INC., West Henrietta , NY, US, 2. September 2004 (2009-09-02), bzw. Dou, Q., J. Elastomers and Plastics, SAGE Publications Ltd. GB, Bd.41, 13. Juli 2009 (2009-07-13), offenbaren Verfahren zur Herstellung von Polypropylene mit einem erhöhten Anteil an β-Propylene. Sie machen aber keinerlei nähereren Angaben zur Herstellung und Eigenschaften der Nukleierungsmittel, insbesondere werden keine Angaben zur allfälligen Verwendung einer nanoskaligen Teilchengrösse gemacht.

Die Aufgabe der vorliegenden Erfindung bestand darin, ein Verfahren zur Erzeugung von β-kristallinem Polypropylen zur Verfügung zu stellen, sowie geeignete β-Nukleierungsmittel mit hoher β-Aktivität. Mittels des Verfahrens sollen hohe β-Antelle reproduzierbar und zuverlässig erreicht werden können. Das Verfahren soll einfach und effizient durchführbar sein. Die Modifizierung den β-Nukleierungsmitteln darf die üblichen wichtigen Gebrauchseigenschaften des Polypropylens nicht beeinträchtigen.

Diese Aufgabe wird gelöst durch ein Verfahren zur Herstellung von Polypropylen mit einem erhöhten Anteil an β-kristallinem Polypropylen, bei dem mindestens ein Me(II)-Dicarbonsäuresalz eines zweiwertigen Nebengruppenmetalls der Formel $Me^{2+}(\text{-OOC-}(CH_2)_x \text{ COO}^-)$, mit x betragend 2 bis 10, und mindestens ein Polypropylen gemischt werden und bei einer Temperatur von mindestens 150°C aufgeschmolzen und anschließend derart abgekühlt werden, daß die abgekühlte Polypropylenschmelze einen erhöhten Anteil von β-kristallinem Polypropylen aufweist.

Dabei wird beim Abkühlen der Polypropylenschmelze ein β-Anteil von mehr als 50 bis <100 Gew.-%, vorzugsweise 70 bis <100 % erzeugt, wobei dieser β-Anteil mittels DSC-Messung an der abgekühlten Schmelze aus der 1. Aufheizkurve bestimmt wird. Ausserdem weist das vorerwähnte Me(II)-Dicarbonsäuresalz eine mittlere Teilchengröße von 1 bis 500nm auf, wobei diese Teilchengröße an einer biaxial verstreckten Folie oder an einer Cast-Folie als Probekörper an einer Rasterelektronischen Mikroskop-Aufnahme der Probe bestimmt wird.

Diese Aufgabe wird auch gelöst durch das besagte Verfahren, wobei die Abkühlung der Polypropylenschmelze in einem Temperaturbereich von 100 - 140°C erfolgt.

Diese Aufgabe wird auch gelöst durch eines der vorstehend angegebenen Verfahren, wobei die Mischung aus Polypropylen und Me(II)-Dicarbonsäuresalz in einem Extruder, vorzugsweise in einem Zweischneckenextruder, bei einer Temperatur von 150 bis 280°C aufgeschmolzen wird.

Diese Aufgabe wird auch gelöst durch eines der vorstehend angegebenen Verfahren, wobei das Polypropylen ein isotaktisches Polypropylen mit einem Schmelzpunkt im Bereich von 140 bis 170°C ist.

Diese Aufgabe wird auch gelöst durch eines der vorstehend angegebenen Verfahren, wobei das Polypropylen ein Mischpolymerisat ist mit einem Comonomeranteil an Ethylen und/oder Butylen von bis zu 10 Gew.-%.

Diese Aufgabe wird auch gelöst durch eines der vorstehend angegebenen Verfahren, wobei das Polypropylen eine Mischung aus Propylenhomopolymer und Propylencopolymer und/oder Propylenterpolymer ist.

Diese Aufgabe wird auch gelöst durch eines der vorstehend angegebenen Verfahren, wobei das Me(II)-Dicarbonsäuresalz eine mittlere Teilchengröße von weniger als 500nm, vorzugsweise 1 bis 30 nm aufweist.

Es ist auch beschrieben, dass das Me(II)-Dicarbonsäuresalz eine mittlere Teilchengröße von mehr als 500nm, vorzugsweise >500nm bis 5$\mu$m aufweist.

Diese Aufgabe wird auch gelöst durch eines der vorstehend angegebenen Verfahren, wobei das Me(II)-Dicarbonsäuresalz ein Adipat oder Pimelat oder Suberat ist.

Diese Aufgabe wird auch gelöst durch eines der vorstehend angegebenen Verfahren, wobei das Me(II)-Dicarbonsäuresalz mit einer Oberflächenbeschichtung versehen ist.

Diese Aufgabe wird auch gelöst durch eines der vorstehend angegebenen Verfahren, wobei die Oberflächenbeschichtung aus langkettigen Fettsäuren, vorzugsweise Ölsäure oder

Stearinsäure, Silanen, Aminen oder Sulfonaten besteht.

Diese Aufgabe wird auch gelöst durch eines der vorstehend angegebenen Verfahren, wobei man eine Mischung aus Polypropylen und Me(II)-Dicarbonsäuresalz herstellt und diese Mischung aufschmilzt und abkühlt.

Diese Aufgabe wird auch gelöst durch eines der vorstehend angegebenen Verfahren, wobei man ein Compound aus Polypropylen und Me(II)-Dicarbonsäuresalz herstellt und dieses Compound mit Polypropylen mischt, aufschmilzt und abkühlt.

Diese Aufgabe wird weiterhin gelöst durch ein Verfahren zur Herstellung einer biaxial verstreckten Flachfolie, bei dem ein Me(II)-Dicarbonsäuresalz eines zweiwertigen Nebengruppenmetalls wie oben beschrieben und Polypropylen gemischt und in einem Extruder bei einer Temperatur von mindestens 150°C aufgeschmolzen und die Schmelze durch eine Flachdüse extrudiert und die Schmelze derart zu einer Vorfolie abgekühlt wird, daß ein Anteil von mindestens 50% $\beta$-kristallinem Polypropylen entsteht, und bei dem die Vorfolie erwärmt und in Längsrichtung und in Querrichtung so verstreckt wird, daß sich das $\beta$-kristalline Polypropylen der Vorfolie in die alpha Modifikation des Polypropylens umwandelt.

Diese Aufgabe wird auch gelöst durch eine Mischung oder Compound aus Polyolefin und mindestens einem Me(II)-Dicarbonsäuresalz eines zweiwertigen Nebengruppenmetalls der Formel $Me^{2+}(^-OOC\text{-}(CH_2)_x\text{-}COO^-)$, wobei das $Me^{2+}$ ein zweiwertiges Metall einer Nebengruppe ist und x 2 bis 10 beträgt, und wobei das Me(II)-Dicarbonsäuresalz eine mittlere Teilchengröße von 1 bis 500nm aufweist.

Es ist auch beschrieben die vorstehend angegebene Mischung oder das vorstehend angegebene Compound, wobei das Me(II)-Dicarbonsäuresalz eine mittlere Teilchengröße von mehr als 500nm, vorzugsweise >500nm bis 5$\mu$m, aufweist.

Diese Aufgabe wird auch gelöst durch eine/s der vorstehend angegebenen Mischungen oder Compounds, wobei das Polyolefin ein isotaktisches Propylenhomopolymer, ein Propylencopolymer, ein Propylenterpolymer oder ein Polyethylen oder eine Mischung aus einem oder mehreren dieser Polyolefine ist.

Diese Aufgabe wird auch gelöst durch eine/s der vorstehend angegebenen Mischungen oder Compounds, wobei das isotaktische Propylenhomopolymer eine Isotaktizität von mindestens 95% aufweist, wobei die Isotaktizität mittels 13C-NMR Spektroskopie (Triade) am n-heptan unlöslichen Anteil des isotaktischen Propylenhomopolymers gemessen wird.

Diese Aufgabe wird auch gelöst durch eine/s der vorstehend angegebenen Mischungen oder Compounds, wobei das isotaktische Propylenhomopolymer eine Isotaktizität von weniger als 95% aufweist, wobei die Isotaktizität mittels 13C-NMR Spektroskopie (Triade) am n-heptan unlöslichen Anteil des isotaktischen Propylenhomopolymers gemessen wird.

Diese Aufgabe wird auch gelöst durch eine/s der vorstehend angegebenen Mischungen oder Compounds, wobei die Mischung zusätzlich Polyolefin, vorzugsgweise Polyethylen, enthält.

Diese Aufgabe wird auch gelöst durch eine/s der vorstehend angegebenen Mischungen oder Compounds, wobei die Mischung oder das Compound einen maximalen $\beta$-Anteil >90% aufweißt, wobei dieser maximale $\beta$-Anteil mittels DSC Messung aus der zweiten Aufheizkurve bestimmt wird

Diese Aufgabe wird auch gelöst durch eine/s der vorstehend angegebenen Mischungen oder Compounds, wobei das Me(II)-Dicarbonsäuresalz eine Me(II)-Pimelat, Me(II)-Adipat oder Me(II)-Suberat ist.

Diese Aufgabe wird auch gelöst durch eine/s der vorstehend angegebenen Mischungen oder Compounds, wobei die Me(II)-Dicarbonsäuresalz mit höherwertigen Carbonsäuren, Silanen, Aminen oder Sulfonaten, vorzugsweise mit Ölsäure oder Stearinsäure beschichtet sind.

Diese Aufgabe wird auch gelöst durch die Verwendung eines Dicarbonsäuresalzes von Me(II)-Dicarbonsäuresalzen der Formel $Me^{2+}(^-OOC\text{-}(CH_2)_x\text{-}COO^-)$, wobei das $Me^{2+}$ ein zweiwertiges Metall einer Nebengruppe ist und x 2 bis 10 beträgt, als $\beta$-Nukleierungsmittel in Polypropylen, wobei das Me(II)-Dicarbonsäuresalz eine mittlere Teilchengröße von 1 bis 500nm aufweist.

Diese Aufgabe wird auch gelöst durch ein Verfahren zur Herstellung von nanoskaligen Me(II)-Dicarbonsäuresalzen der Formel $Me^{2+}(^-OOC\text{-}(CH_2)_x\text{-}COO^-)$, wobei das $Me^{2+}$ ein zweiwertiges Metall einer Nebengruppe ist und x 2 bis 10 beträgt, bei dem eine wässrige Lösung einer Dicarbonsäure hergestellt wird und dieser wässrigen Lösung eine Me(II)SalzLösung oder ein Me(II)-Salz hinzugefügt wird und das ausgefallene Dicarbonsäuresalz eines zweiwertigen Nebengruppenmetalls abgetrennt und getrocknet wird und das getrocknete Dicarbonsäuresalz eines zweiwertigen Nebengruppenmetalls in einem nicht-wässrigen flüssigen Medium aufgeschlämmt wird und die Schlämme zur Zerkleinerung der aufgeschlämmten Dicarbonsäuresalz eines zweiwertigen Nebengruppenmetalls bearbeitet wird, wobei das Me(II)-Dicarbonsäuresalz eine mittlere Teilchengröße von 1 bis 500nm aufweist.

Die abhängigen Unteransprüche geben bevorzugte Ausführungsformen der Erfindung an.

Die vorliegende Erfindung beruht auf der Entdeckung, daß Dicarbonsäuresalze eines zweiwertigen Nebengruppenmetalls (nachstehend auch Me(II)-Dicarbonsäuresalze) beim Abkühlen einer Polypropylen-Schmelze, die mindestens ein Me(II)-Dicarbonsäuresalz enthält, zur Bildung eines hohen Anteils von β-kristallinem Polypropylen führen.

Me(II)-Dicarbonsäuresalze [$Me^{2+}(^-OOC-(CH_2)_x-COO^-)$] sind Salze der zweiwertigen

Nebengruppenmetalle. Dicarbonsäuren sind gesättigte aliphatische Dicarbonsäuren, die der allgemeinen Formel $HOOC-(CH_2)_x-COOH$ entsprechen, worin X 2 bis 10 beträgt. Bevorzugte Dicarbonsäuren sind Pimelinsäure (1,6-Hexandisäure mit X=4); Adipinsäure (1,5-Pentandicarbonsäure mit X=3) und Suberinsäure (1,7-Heptandisäure mit X=5). Zweiwertige Nebengruppenmetalle sind beispielsweise Nickel, Eisen, Ruthenium, Osmium, Kupfer, Silber, Gold, Cobalt, Zink, Cadmium, Quecksilber, Rhodium, Iridium, Palladium, Scandium, Hafnium, Titan, Vanadium, Niob, Tantal, Wolfram, Mangan Technecium, Rhenium, Platin, Palladium, Zinn oder Blei sowie die Elemente der Lanthangruppe und der Actiniumgruppe alle in ihrer zweiwertigen Form. Zweiwertige Nebengruppenmetalle werden nachstehend auch als Me(II) abgekürzt.

Die Synthese der Me(II)-Dicarbonsäuresalze erfolgt über die an sich bekannte Fällungsreaktion der aliphatischen Dicarbonsäuren, z.B. Pimelinsäure, Adipinsäure oder Suberinsäure, mit zweiwertigen Nebengruppenmetallsalzen, wie beispielsweise Chloriden, Carbonaten oder Hydroxiden, da die Bildung von Salzsäure oder CO2 als Nebenprodukt vermieden wird. Für die Umsetzung wird im allgemeinen eine wäßrige Lösung der aliphatischen Dicarbonsäure vorgelegt. Die aliphatische Dicarbonsäure wird in Wasser gegeben und unter Rühren erwärmt bis sich die aliphatische Dicarbonsäure gelöst hat, beispielsweise bei einer Temperatur von 70 bis 95°C, vorzugsweise 75 bis 90°C. Anschließend wird zu dieser Dicarbonsäure-Lösung unter weiterem Rühren das Me(II)-Salz, vorzugsweise Ni(OH)2 direkt oder eine wässrige Me(II)salz-Lösung, vorzugsweise eine Ni(OH)2-Lösung, hinzugefügt. Die Reaktanten werden hierbei im allgemeinen in stöchiometrischen Mengen eingesetzt. Dabei fällt das Me(II)-Dicarbonsäuresalz als feiner Niederschlag aus. Dieser Niederschlag sedimentiert, wird abgetrennt und durch geeignete Verfahren getrocknet, z.B. in einem Trockenschrank, bei 100 - 120°C vorgetrocknet. Anschließend wird, beispielsweise unter Vakuum, z.B. in einem Vakuumtrockenschrank, bei ca. 150 bis 200°C der Restfeuchtegehalt des Me(II)-Dicarbonsäuresalzes weiter gesenkt. Vorzugsweise beträgt der Wassergehalt des getrockneten Me(II)-Dicarbonsäuresalzes 0 - 2 Gew. %, vorzugsweise >0 bis 1 Gew.-%. Auf diese Weise wird ein trockenes, pulverförmiges Me(II)-Dicarbonsäuresalz erhalten.

Für die Herstellung von nanoskaligen Me(II)-Dicarbonsäuresalzen mit einer Partikelgröße von unter 500nm sind auch die vorstehend beschriebenen Umsetzungen von Me-(II)-Salzen mit der jeweiligen Dicarbonsäure im wässrigen Medium und anschließender Trocknung geeignet. Nanoskalige Me(II)-Dicarbonsäuresalze haben im allgemeinen eine Teilchengröße von 1 bis 500nm, vorzugsweise 5 bis 300nm, wobei gleichzeitig Teilchen oder Agglomerate mit einer Teilchengröße von >1 $\mu$m zu weniger als 3%, vorzugsweise >0 bis <1% enthalten sind. Somit liegt auch die mittlere Teilchengröße der nanoskaligen Me(II)-Dicarbonsäuresalze im besagten Bereich von 1 bis 500nm, vorzugsweise 5 bis 300nm. Entsprechend beträgt die Teilchengröße von nicht-nanoskaligen Me(II)-Dicarbonsäuresalzen >500nm, vorzugsweise 700nm bis 5$\mu$m, insbesondere 800nm bis 3$\mu$m.

Zur Herstellung der nanoskaligen Me(II)-Dicarbonsäuresalze wird das trockene pulverförmige Me(II)-Dicarbonsäuresalz nach Fällung und Trocknung in einem nicht-wässrigen flüssigen Medium aufgeschlämmt und z.B. durch Vermahlen der Schlämme, bearbeitet Im allgemeinen werden mindestens 5 bis 60 Gew.-%, vorzugsweise 10 bis 50 Gew.-% insbesondere 15 bis 40 Gew.-%, bezogen auf das Gewicht der nicht-wässrigen flüssigen Phase, des Me(II)-Dicarbonsäuresalzes in der nicht-wässrigen flüssigen Phase aufgeschlämmt. Dieser Schlamm wird anschließend vermahlen. Zur Zerkleinerung dienen beispielsweise gängige Mörsermühlen, Ultraschall oder eine Kugelmühle oder andere gängige Nassmahl- oder Zerkleinerungsprozesse. Hierbei wird das Me(II)-Dicarbonsäuresalz auf eine Partikelgröße von unter 1 bis 500nm, insbesondere 5 bis 300nm zerkleinert. Das nanoskalige Me(II)-Dicarbonsäuresalz bildet nach der Zerkleinerung in der nicht-wässrigen flüssigen Phase eine stabile Dispersion in der Agglomerate von mehr als 1000nm nur in geringen Mengen oder gar nicht mehr vorliegen. Der Übergang zu der nanoskaligen dispergierten Phase zeigt sich auch darin, daß das aufgeschlämmte Me(II)-Dicarbonsäuresalz vor der Mahlung zunächst direkt, z.B. innerhalb weniger Minuten, wieder sedimentiert, aber nach der Vermahlung eine stabile, milchige, trübe Dispersion bildet, in der sich die Teilchen nicht mehr absetzen. Diese Dispersion ist für eine übliche Zeitdauer bis zur Verarbeitung im wesentlichen stabil, z.B. für eine Dauer von mindestens einer oder auch mehreren Stunden. Gegebenenfalls kann die Dispersion zusätzlich filtriert werden, um Agglomerate abzutrennen, die nach der Mahlung noch vorhanden sind. Das Filtermedium wird so gewählt, daß alle Teilchen mit einer Größe von >1$\mu$m abgetrennt werden und die Dispersion anschließend frei von Teilchen dieser Größe ist oder zumindest davon weniger als 1 % enthält.

Unter einem nicht-wässrigen flüssigen Medium oder einer nicht-wässrigen flüssigen Phase wird im Sinne der vorliegenden Erfindung eine organische Verbindung verstanden, die bei Raumtemperatur flüssig ist und deren Wassergehalt <1Gew.-% beträgt, beispielsweise Alkohole, niedere Alkane, Ketone und ähnliche Flüssigkeiten. Bevorzugt sind Hexan, Heptan oder Ethanol, Butanol oder Isopropanol oder Aceton. Es können auch Mischungen dieser flüssigen Phasen verwendet werden

Trocknung bedeutet, je nach gegebenem Zusammenhang, im Sinne der vorliegenden Erfindung sowohl die Entfernung von Wasser oder Feuchte als auch die Abtrennung des nicht-wässrigen, flüssigen Mediums.

Die Dispersion kann entweder direkt mit dem Polypropylen, z.B. in Form von Pulver oder Granulat, vermischt, beispielsweise durch Auftrommeln, und getrocknet werden. Alternativ wird die nicht-wässrige flüssige Phase der Dispersion abgetrennt und das so erhaltene Pulver aus nanoskaligem Me(II)-Dicarbonsäuresalz mit Polypropylen, in Form von Pulver oder Granulat, vermischt. Über beide mögliche Verfahrensvarianten erhält man eine Mischung aus nanoskaligen Me(II)-Dicarbonsäuresalzen und Polypropylen. Die Abtrennung der nicht-wässrigen flüssigen Phase erfolgt in beiden Verfahren mit üblichen geeigneten Mitteln, beispielsweise durch Verdunsten, Abziehen im Vakuum, Abdestillieren oder mittels einer Filterpresse. Die Mischungen enthalten im allgemeinen 0 bis 2 Gew.-% der flüssigen Phase, vorzugsweise >0 bis 1 Gew.-%.

Alle vorstehend beschriebenen Me(II)-Dicarbonsäuresalze werden nach der Trocknung mit üblichen Verfahren mit dem Polypropylen vermischt oder compoundiert. Hierzu werden beispielsweise mechanische Vormischungen aus Polypropylengranulat und Me(II)-Dicarbonsäuresalz, gegebenenfalls als Dispersion, hergestellt. Vorzugsweise wird aus Me(II)-Dicarbonsäuresalz und Polypropylen ein Compound hergestellt. Unter einem Compound wird im Sinne der vorliegenden Erfindung eine homogene Mischung aus mindestens einem Polypropylen und Me(II)-Dicarbonsäuresalz als Additiv verstanden. Die Herstellung des Compounds erfolgt in üblicher Weise in dem die Vormischung oder Polypropylen und Me(II)-Dicarbonsäuresalz bei geeigneten Temperaturen aufgeschmolzen wird, beispielsweise in einem Bereich von 160 bis 300°C. Das Aufschmelzen erfolgt vorzugsweise in einem geeigneten Extruder, beispielsweise in einem Zweischneckenextruder, welcher gleichzeitig eine gute Mischung des Me(II)-Dicarbonsäuresalzes im Polypropylen gewährleistet. Die aufgeschmolzene Mischung wird zu Granulatkörnern extrudiert und diese bei geeigneten Temperaturen abgekühlt. Bei der Compoundierung können zusätzlich zu dem Polypropylen weitere Zusatzstoffe und/oder andere Polyolefine hinzugefügt werden, beispielsweise Polyethylene.

Die Mischung oder das Compound aus Polypropylen und Me(II)-Dicarbonsäuresalz enthält im allgemeinen mindestens 85 Gew.-%, vorzugsweise 95 bis <100 Gew.-%, insbesondere 97 bis <100 Gew.-%, eines Polypropylens, sowie gegebenenfalls weitere Zusatzstoffe und 0,0001 bis 5 Gew.-%, vorzugsweise 0,001 bis 3 Gew.-% Me(II)-Dicarbonsäuresalz. Insbesondere für Folienanwendungen ist ein Gehalt von 0,001 bis 2 Gew.-% Me(II)-Dicarbonsäuresalze in dem Compound oder der Mischung bevorzugt. Die Angaben in Gew.-% beziehen sich jeweils auf das Gewicht der Mischung oder des Compounds. Gegebenenfalls können auch verschiedene Me(II)-Dicarbonsäuresalz gemischt werden. Des weiteren können die Me(II)-Dicarbonsäuresalz auch zusammen mit weiteren an sich bekannten β-Nukleierungsmitteln eingesetzt werden.

Gegebenenfalls kann zusätzlich zur Vermeidung von Agglomeration, insbesondere bei den nanoskaligen Me(II)-Dicarbonsäuresalzen, und zur Verbesserung der Dispergierbarkeit der Me(II)-Dicarbonsäuresalze in der Polypropylen-Matrix eine oberflächenaktive Substanz, wie z.B. höherwertige Carbonsäuren, Silane, Amine oder Sulfonate zugesetzt werden. Besonders bevorzugt sind für diese Zwecke langkettige Fettsäuren, wie Ölsäure oder Stearinsäure. Der Zusatz dieser Hilfsstoffe erfolgt beispielsweise beim Vermischen der Me(II)-Dicarbonsäuresalze mit Polypropylen oder bei der Herstellung der Dispersion oder beim Vermischen der Dispersion mit Polypropylen. Derartige Verfahren sind an sich im Stand der Technik bekannt, beispielsweise in Macromol. Mater. Eng. 275, 8-17 (2000) sowie in GAK 5/1988 Jahrgang 41, Seite 211 ff oder Macromol. Rapid Commun, 2001, 22, 176-180 beschrieben. Derartige Beschichtungen werden bevorzugte für nanoskalige Me(II)-Dicarbonsäuresalze verwendet.

Die erfindungsgemäßen Mischungen oder Compounds aus Polypropylen und Me(II)-Dicarbonsäuresalz können direkt zur Herstellung von Produkten, beispielsweise Spritzgußteilen, Folien, poröse Folien, Fasern etc. eingesetzt oder zusammen mit weiteren Polyolefinen und/oder weiteren Zusatzstoffen verarbeitet werden.

Polypropylene umfassen im Sinne der vorliegenden Erfindung Propylenhomopolymere und Propylenmischpolymerisate mit einem oder mehreren olefinischen Comonomeren mit 2 oder 4 bis 10 C-Atomen. Im allgemeinen enthalten die Propylenpolymeren 70 bis 100 Gew.-%, vorzugsweise 80 bis 99 Gew.-%, insbesondere 90 bis 98 Gew.-%, Propylen. Der entsprechende Comonomergehalt von höchstens 30 Gew.-% bzw. 1 bis 20 Gew.-% bzw. 2 bis 10 Gew.-% besteht, wenn vorhanden, im allgemeinen aus Ethylen und/oder Butylen. Die Angaben in Gew.-% beziehen sich jeweils auf das Propylenpolymere. Geeignete Mischpolymerisate, welche vorzugsweise Ethylen und/oder Butylen als Comonomer enthalten, sind statistische Mischpolymerisate oder Blockcopolymere. Bevorzugt sind auch isotaktische Propylenhomopolymere mit einem Schmelzpunkt von 140 bis 170°C, vorzugsweise von 155 bis 165°C, und einen Schmelzflußindex (Messung DIN 53 735 bei 21,6 N Belastung und 230°C) von 1,0 bis 50 g/10 min, vorzugsweise von 1,5 bis 20 g/10 min.

Der n-heptanlösliche Anteil der Polypropylene beträgt im allgemeinen 1 bis 10 Gew.-%, vorzugsweise 2-5 Gew.-% bezogen auf das Ausgangspolymere. Die Molekulargewichtsverteilung der Polypropylene kann variieren. Das Verhältnis des Gewichtsmittels $M_w$ zum Zahlenmittel $M_n$ liegt im allgemeinen zwischen 1 und 15, vorzugsweise bei 2 bis 10, ganz besonders bevorzugt bei 2 bis 6. Eine derartig enge Molekulargewichtsverteilung der Polypropylene erreicht man beispielsweise durch dessen peroxidischen Abbau oder durch Herstellung des Polypropylens mittels geeigneter Metallocenkatalysatoren.

In einer weiteren Ausführungsform der Erfindung ist das eingesetzte Polypropylen, insbesondere Propylenhomopolymere hochisotaktisch. Für derartige hochisotaktische Polypropylene beträgt der mittels $^{13}$C-NMR-Spektroskopie bestimmte Kettenisotaxie-Index (Triade) des n-heptanunlöslichen Anteils des Polypropylens mindestens 95 %, vorzugsweise 96 bis 99 %.

Nach dem erfindungsgemäßen Verfahren zur Herstellung von Polypropylen mit einem erhöhten Anteil an β-kristallinem Polypropylen wird zunächst eine Mischung oder ein Compound aus Polypropylen und Me(II)-Dicarbonsäuresalz bei geeigneten Temperaturen aufgeschmolzen. Das Aufschmelzen erfolgt vorzugsweise in einem geeigneten Extruder, beispielsweise in einem Zweischneckenextruder, welcher gleichzeitig eine gute Verteilung der Me(II)-Dicarbonsäuresalze im Polypropylen gewährleistet. Die Extrudertemperatur liegt dabei im allgemeinen in einem Bereich von 180 bis 280°C. Die aufgeschmolzene Mischung wird extrudiert und bei geeigneten Temperaturen abgekühlt. In anderen Verfahrensvarianten erfolgt die Herstellung der Mischung bzw. des Compounds wie vorstehend beschrieben in einem vorgelagerten Arbeitsschritt. Diese Mischungen oder Compounds werden anschließend in dem erfindungsgemäßen Verfahren zusammen mit einem weiteren Polypropylen eingesetzt, welches das gleiche Polypropylen wie in der Mischung/Compound oder ein davon verschiedenes Polypropylen sein kann. Die Mischungen oder Compounds können in einem beliebigen Extrusionswerkzeug oder in einem Kneter aufgeschmolzen und mit dem weiteren Polypropylen gemischt werden.

Es ist erfindungswesentlich, daß nach der Extrusion die Abkühlung der salzhaltigen Polypropylen-Schmelze derart erfolgt, daß die β-nukleierende Wirkung der Me(II)-Dicarbonsäuresalze zum Tragen kommt. Hierfür ist es bevorzugt die Schmelze langsam bei einer Temperatur in einem Bereich von 60 bis 140 °C, vorzugsweise bei 80 bis 130°C abzukühlen. Je näher diese Temperatur in der Nähe der Kristallisationstemperatur des β-kristallinen Polypropylens (<140°C) liegt, umso günstiger sind die Bedingungen für die Ausbildung der β-kristallinen Modifikation. Auf diese Wiese kann über die Auswahl der Temperatur beim Abkühlen ein mehr oder weniger hoher Anteil an β-Polypropylen erzeugt werden. Zusätzlich hat die Verweildauer der abkühlenden Schmelze bei der jeweiligen Temperatur einen Einfluß auf den erzielten β-Anteil. Zur Erzielung eines größtmöglichen β-Anteils sollte die Schmelze langsam bei höheren Temperaturen (120-140°C) abgekühlt werden, wobei die notwendige Verweildauer bei der gegebenen Temperatur im Einzelfall auch von der Formgebung bei der Extrusion abhängt.

Je nach Anwendungsfall können auch niedrigere β-Anteile, wie beispielsweise 10 bis 50%, im Polypropylen ausreichend sein. Die β-nukleierenden Me(II)-Dicarbonsäuresalze wirken sich in diesen Fällen positiv aus, da die Abkühlrate erhöht werden kann, d.h. schnellere Abzugsgeschwindigkeiten eingesetzt werden können.

Mittels des erfindungsgemäßen Verfahrens ist es möglich bei entsprechenden Abkühlbedingungen einen Gehalt an β-Polypropylen von >50 bis <100 Gew.-%, vorzugsweise 70 bis 99 Gew.-%, insbesondere 90 bis 99% (DSC-Methode, 1.Aufheizkurve) zu erzielen. Beispielsweise wurden über DSC-Messungen (1.Aufheizkurve, Methode nachstehend beschrieben) an isotaktischem Propylenhomopolymer mit 0,2 Gew.-% Me(II)-Dicarbonsäuresalz ein Anteil von β-kristallinem Polypropylen von bis zu 98,5 % bestimmt.

Das erfindungsgemäße Verfahren kann vorteilhaft bei der Herstellung von Folien, Formkörpern, insbesondere Rohren und Schläuchen, Fasern und anderen Extrusionsprozeße angewendet werden. Der erhöhte β-Anteil im Polypropylen wirkt sich bei den verschiedensten Extrusionsanwendungen günstig aus, beispielsweise da die Extrusionstemperaturen reduziert werden können. Für einige Anwendungen ist ein erhöhter Anteil an β-kristallinem Polypropylen vorteilhaft, da hierdurch Gebrauchseigenschaften des Polypropylens verbessert werden, z.B. erreicht man eine höhere Kerbschlagzähigkeit und Spannungsrißbeständigkeit des Polypropylens. In einer weiteren Anwendung nutzt man den hohen β-Anteil im Polypropylen zur Herstellung von porösen Folien durch Umwandlung der β-Modifikation in die alpha-Modifikation bei der Verstreckung von Folien oder zur Erzeugung von rauhen Oberflächen einer verstreckten Folie aus.

Grundsätzlich können in dem erfindungsgemäßen Verfahren alle vorstehend beschriebenen Polypropylene, d.h. Propylenehomopolymere und/oder Propylencopolymere und/oder Propylenterpolymere, verwendet werden. Im allgemeinen ist es bevorzugt Propylenhomopolymere einzusetzen.

Es wurde gefunden, daß die Me(II)-Dicarbonsäuresalze auch sehr vorteilhaft in einem Verfahren zur Herstellung einer porösen, vorzugsweise biaxialen verstreckten, Folie oder auch einer Folie mit einer oder mehreren porösen Schichten eingesetzt werden können. Die hohen Gehalte an β-Polypropylen wirken sich positiv auf die Porosität dieser Folie, bzw. der porösen Schicht, und deren Gasdurchlässigkeit aus. Bei Verwendung der erfindungsgemäßen Me(II)-Dicarbonsäuresalze können Streckbedingungen, insbesondere hohe Streckfaktoren angewendete werden, die zu einer besonders hohen Porosität der Folie, bzw. der Schicht führen, wobei gleichzeitig eine überraschend gute Laufsicherheit der Folie gegeben ist. Gegebenenfalls kann die Erfindung auch bei einer mehrschichtigen Folie genutzt werden, die neben einer oder mehreren porösen Schicht/en auch eine weitere oder mehrere im wesentlichen gas-undurchlässige Schicht/en, beispielsweise aus Polyolefinen umfaßt. Die Angaben in dieser Beschreibung bezüglich der porösen Folie gelten somit sinngemäß in gleicher Weise oder analog auch für die poröse Schicht oder die porösen Schichten einer mehrschichtigen Folie.

Im Einzelnen werden bei der Herstellung einer biaxial verstreckten porösen Polypropylenfolie alle Komponenten der porösen Schicht oder Schichten in einem oder mehreren Extruder bei einer Temperatur von mindestens 160°C aufgeschmolzen. Die ein-oder mehrschichtige Polymerschmelze wird durch eine Flachdüse co/extrudiert, von einer Abnahmewalze aufgenommen und auf der Abnahmewalze derart abgekühlt, daß sich die Schmelze zu einer Vorfolie verfestigt und der gewünschte Anteil an β-kristallinem Polypropylen entsteht. Diese Abkühlung der Schmelze erfolgt vorzugsweise in einem Temperaturbereich von 80 bis 130°C, wobei eine lange Verweildauer bei dieser Temperatur zu einem erhöhten β-Polypropylen-Anteil beiträgt. Für die Herstellung einer porösen Folien, bzw. Schicht wird im allgemeinen ein Anteil von mindestens 40%, vorzugsweise 60 bis 98% an β-Polypropylen in der Vorfolie (gemessen nach DSC, 1.Aufheizkurve) angestrebt, wohingegen zur Erzeugung von Oberflächenrauhigkeiten geringere Anteile von beispielsweise 10 bis 40% ausreichend sein können. Anschließend wird die Vorfolie in an sich bekannter Weise erwärmt und in Längsrichtung verstreckt, vorzugsweise bei einer Temperatur von weniger als 140°C, insbesondere 80 bis 125°C und mit einem Streckfaktor von 2,5:1 bis 6:1. Nach der Längsstreckung wird die längsgestreckte Folie erneut erwärmt und in Querrichtung verstreckt, vorzugsweise bei einer Temperatur größer 110°C, insbesondere von 120 bis 145°C und mit einem Streckverhältnis von 3:1 bis 8:1. Durch die gewählten Temperaturen bei der Verstreckung wandelt sich das β-kristalline Polypropylen der Vorfolie in die alpha Modifikation des Polypropylens um und erzeugt je nach Verfahrensbedingungen und β-Anteil in der Vorfolie eine durchgehende poröse netzwerkartige Struktur in der Folie, bzw. in der porösen Schicht oder zumindest eine Oberflächenrauhigkeit durch kraterartige Vertiefungen, die bei den Umwandlungsprozeßen entstehen. Derartige rauhe Oberflächenstrukturen sind beispielsweise bei Folien mit papierähnlichem Charakter oder bei Kondensatorfolien erwünscht, welche als Dielektrikum in Kondensatoren eingesetzt werden. Um die elektrischen Eigenschaften solcher Kondensatorfolien nicht zu beeinträchtigen ist es bevorzugt das Me(II)-Dicarbonsäuresalze nur in der oder den Deckschicht/en einzusetzen, welche die Oberflächenrauheit aufweisen sollen. Es wurde gefunden, daß die, insbesonderen, nano-skaligen Me(II)-Dicarbonsäuresalze die elektrischen Eigenschaften der Kondensatorfolie nicht oder nur geringfügig beeinträchtigen.

Überraschenderweise hat die Folie, bzw. die Schicht, welche die erfindungsgemäßen Me(II)-Dicarbonsäuresalzen enthält, eine sehr hohe und gleichmäßige Porosität und eine gute mechanische Festigkeit auf. Die gleichmäßige Verteilung der Porengröße ist in REM-Aufnahmen gut zu erkennen. Der mittlere Porendurchmesser (Bubble Point) liegt im Bereich von 10 bis 350nm, bevorzugt im Bereich 40 bis 300nm. Bei der Herstellung der porösen Folie, bzw. der Folie mit poröser Schicht, kommt es nur sehr selten zu Abrissen, d.h. das Verfahren hat eine hohe Laufsicherheit. Die Folie kann mit sehr hohen Faktoren verstreckt werden, so daß außergewöhnlich hohe Porositäten erzielt werden können. Grundsätzlich kann der Gurley-Wert der verschiedenen Ausführungsformen der porösen Folie in einem breiten Bereich variieren. Für solche Folien, welche nur poröse Schichten umfassen und beispielsweise als Membran-Folien verwendet werden, liegt der Gurley Wert im allgemeinen in einem Bereich von 100 - 5000s; vorzugsweise 100 bis 2000s. Überraschenderweise lassen sich nach der vorliegenden Erfindung durch hohe Streckfaktoren auch poröse Folien mit sehr niedrigen Gurley Werten von 10 bis <100s, vorzugsweise 15 bis 80s, insbesondere 15 bis 50s noch verfahrenssicher herstellen. Auch lassen sich poröse Folien mit Gurley Werten <600s und Porositäten >50% mit einer Dicke unter 30 μm, vorzugsweise 10 - 25μm, insbesondere 12 - 20μm noch laufsicher herstellen.

Die porösen Folie, bzw. die poröse/n Schicht/en der Folie enthält/enthalten in einer weiteren Ausführungsform Me(II)-Dicarbonsäuresalzen und isotaktisches Propylenhomopolymer und Propylenblockcopolymer, sowie gegebenenfalls weitere Polyolefine, welche die Porosität nicht beeinträchtigen. In diesen Ausführungsformen enthält die Folie bzw. die poröse Schicht im allgemeinen 50 bis 85 Gew.-%, vorzugsweise 60 bis 75 Gew.-%, Propylenhomopolymere und 15 bis 50 Gew.-% Propylenblockcopolymere, vorzugsweise 25 bis 40 Gew.-%, und 0,001 bis 5 Gew.-%, vorzugsweise 50 - 10.000 ppm des Me(II)-Dicarbonsäuresalzes als β-Nukleierungsmittels, bezogen auf das Gewicht der porösen Schicht, bzw. bezogen auf das Gewicht der Folie. Gegebenenfalls sind zusätzlich übliche Additive in geringen Mengen von unter 2 Gew.-%, beispielsweise Stabilisatoren und Neutralisationsmittel enthalten. Für den Fall, daß weitere Polyolefine enthalten sind, wird der Anteil des Propylenhomopolymeren oder des Blockcopolymeren entsprechend reduziert. Weitere Polyolefine sind beispielsweise Polyethylene, wie HDPE oder MDPE. Im allgemeinen sind diese Polyethylene wie HDPE und MDPE mit dem Polypropylen unverträglich und bilden im Gemisch mit Polypropylen eine separate Phase, die sich in einer DSC Messung durch einen separaten Schmelzepeak im Bereich der Schmelztemperatur des Polyethylens zeigt. HDPE hat im allgemeinen Schmelzpunkt, gemessen mit DSC (Maximum der Schmelzkurve, Aufheizgeschwindigkeit 20 °C/min), liegt zwischen 120 und 145°C, vorzugsweise 125 - 140°C, einen MFI (50 N/190 °C) von größer 0,1 bis 50 g/10 min, vorzugsweise 0,6 bis 20 g/10min, gemessen nach DIN 53 735 und eine Kristallinität von 35 bis 80 %, vorzugsweise 50 bis 80 % und eine Dichte, gemessen bei 23 °C nach DIN 53 479, Verfahren A, oder ISO 1183, von >0,94 bis 0,97 g/cm3. MDPE hat im allgemeinen einen Schmelzpunkt, gemessen mit DSC (Maximum der Schmelzkurve, Aufheizgeschwindigkeit 20 °C/min), zwischen 115 und 130°C, vorzugsweise 120 - 125°C, einen MFI (50 N/190 °C) von größer 0,1 bis 50 g/10 min, vorzugsweise 0,6 bis 20 g/10min, gemessen nach DIN 53 735, eine Dichte, gemessen bei 23°C nach DIN 53 479, Verfahren A, oder ISO 1183, von >0,925 bis 0,94 g/cm$^3$.

Im allgemeinen wird die Menge der zusätzlichen Polymeren 0 bis <50 Gew.-%, vorzugsweise 0,5 bis 40 Gew.-%, insbesondere 1 bis 30 Gew.-% betragen, wenn diese zusätzlich enthalten sind. In diesen Fällen wird der vorstehend beschriebene Anteil an Polypropylenen oder Propylenblockcopolymeren entsprechend erniedrigt. In gleicher Weise gilt, daß der besagte Propylenpolymer- oder Propylenblockcopolymer-Anteil reduziert wird, wenn höhere Mengen von bis zu 2 Gew.-% Nukleierungsmittel eingesetzt werden.

Die poröse Folie kann einschichtig oder mehrschichtig sein. Die Dicke der porösen Folie liegt im allgemeinen in einem Bereich von 10 bis 200$\mu$m, vorzugsweise 15 bis 150$\mu$m, insbesondere 15 bis 100$\mu$m. Die Dichte der porösen Folie liegt im allgemeinen in einem Bereich von 0,1 bis 0,6 g/cm3, vorzugsweise 0,2 bis 0,5 g/cm3. Die poröse Folie kann mit einer Corona, Flamm- oder Plasmabehandlung versehen werden, um die Befüllung mit Elektrolyten zu verbessern. Gegebenenfalls kann die mikroporöse Folie eine Abschaltschicht umfassen, welche die Durchlässigkeit der Folie bei erhöhten Temperaturen herabsetzt.

Die porösen Folien können vorteilhaft als Membran verwendet werden, beispielsweise in Batterien, sekundären Batterien, in Superkondensatoren oder in ähnlichen Anwendungen.

Zur Charakterisierung der Rohstoffe und der Folien wurden die folgenden Meßmethoden benutzt:

Schmelzflußindex

Der Schmelzflußindex der Propylenpolymeren wurde nach DIN 53 735 bei 2,16 kg Belastung und 230 °C gemessen und bei 190°C und 2,16 kg für Polyethylene.

Schmelzpunkte

Bei der DSC-Messung wird dem Polymeren mit einer definierten Aufheizrate eine Wärmemenge pro Zeiteinheit zugeführt und der Wärmestrom gegen die Temperatur aufgetragen. Der Schmelzpunkt ist im Sinne der vorliegenden Erfindung das Maximum der DSC Kurve. Zur Bestimmung des Schmelzpunkts wird die DSC-Kurve mit einer Aufheiz- und Abkühlgeschwindigkeit von 10K/1min im Bereich von 20 bis 200°C aufgenommen. Für die Bestimmung des Schmelzpunkts der Polymeren wird wie üblich die zweite Aufheizkurve ausgewertet.

Dichte

Die Dichte $\rho$ wird nach DIN 53 479, Verfahren A, bestimmt.

Porosität

Die Porosität errechnet sich aus der an der porösen Folie bestimmten Dichte $\rho$F und der Dichte des Ausgangsrohstoffes Polyproylen wie folgt:

$$P\,[\%] = 100 \times (1 - \rho F / \rho PP)$$

Dabei wurde für Polypropylen eine Dichte von 0,92g/cm$^3$ angenommen.

Permeabilität (Gurley-Wert)

Die Permeabilität der Folien wurde mit dem Gurley Tester 4110, nach ASTM D 726-58 gemessen. Dabei wird die Zeit (in sec) bestimmt die 100 cm$^3$ Luft benötigen, um durch eine Fläche von 1 Inch$^2$ (6,452 cm$^2$) zu permeieren. Die Druckdifferenz über der Folie entspricht dabei dem Druck einer Wassersäule von 12,4 cm Höhe. Die benötigte Zeit entspricht dann dem Gurley-Wert.

$\beta$-Gehalt

Der Anteil des $\beta$-kristallinen Polypropylens wird mittels DSC bestimmt. Die mit dem Me(II)-Dicarbonsäuresalz additivierte Probe (Mischung, Compound, Folie, Formkörper) wird in der DSC zunächst mit einer Aufheizrate von 10K/min auf 220°C erhitzt und aufgeschmolzen (1. Aufheizen). Danach wird sie mit einer Kühlrate von 10K/min auf 100°C abgekühlt, bevor sie mit einer Heizrate von 10K/min (2. Aufheizen) wieder aufgeschmolzen wird.

Aus der DSC Kurve des 1. Aufheizen wird aus dem Verhältnis der Schmelzenthalpien der $\beta$-kristallinen Phase (H$\beta$) zu der Summe der Schmelzenthalpien von $\beta$- und $\alpha$-kristalliner Phase (H$\beta$ + H$\alpha$) der Anteil an $\beta$-kristallinem Polypropylen der Probe bestimmt, der in der vermessenen Probe vorliegt (unverstreckte Folie, Formkörper, Spritzgussteil). Der prozentuale Wert errechnet sich wie folgt:

$$K\beta,DSC\ [\%] = 100 \times (H\beta)/(H\beta + H\alpha)$$

Aus der DSC Kurve des 2. Aufheizen wird aus dem Verhältnis der Schmelzenthalpien der β -kristallinen Phase (Hβ) zu der Summe der Schmelzenthalpien von β- und α-kristalliner Phase (Hβ + Hα) der Kristallinitätsgrad Kβ,DSC (2. Aufheizen) bestimmt, der den β-Anteil der jeweiligen Polypropylenprobe angibt, der maximal erreicht werden kann.

Agglomerate und Teilchengröße

Die Teilchengröße der Me(II)-Dicarbonsäuresalze und das Vorliegen von Agglomeraten wird an Rasterelektronischen Mikroskop-Aufnahme (REM) der Proben bestimmt.
Zur Durchführung der REM-Aufnahmen an einer Folienprobe wird ein Zuschnitt von 5x5mm aus der biaxial verstreckten Folie ausgeschnitten und auf den Probenträger aufgeklebt. Anschließend wird in einer Sputtereinheit eine wenige Nanometer dicke Schicht eines Edelmetalls (Pt, Au, Pd..) auf die Oberfläche der Folie aufgebracht.
Die besputterte Probe wird dann über eine Schleuse in das REM eingebracht und dort im Hochvakuum mit einer Beschleunigungsspannung von mehreren kV abgescannt. Die Beschleunigungsspannung wird so gewählt, dass ein scharfes Bild entsteht, ohne daß sich die Folien-Matrix auf Grund thermischer Belastung deformiert. Die Teilchen sind in der Aufnahme so gut zu erkennen, daß die Größe der einzelnen Partikel mit Hilfe des Maßstabs ausgemessen werden kann.
Die entsprechende Bestimmung der Teilchengröße der Me(II)-Dicarbonsäuresalze im Compound erfolgt an einer Cast-Folie als Probekörper. Hierfür wird aus dem Compound eine ca. 120 bis 150μm unverstreckte Castfolie hergestellt. Mit dieser Cast-Folie wird verfahren wie oben beschrieben.
Die Folie, bzw. das Compound ist im Sinne der vorliegenden Erfindung frei von Agglomeraten, wenn in der REM-Aufnahme der Folien-Probe keine Teilchen mit einer Größe von mehr als einem 1μm gefunden werden oder wenn maximal ein Teilchen von >1μm vorhanden ist. Die mittlere Teilchengröße kann durch Ausmessen der Teilchengröße einer statisch ausreichenden Anzahl von Teilchen erfolgen. Entsprechend kann auch der Anteil an Agglomeraten von >1μm an Hand der REM Aufnahmen bestimmt werden.
Die Erfindung wird nun an Hand von Ausführungsbeispielen näher erläutert:

Beispiele 1: Herstellung von Nickelpimelat

Beispiel 1a :(Vergleichsbeispiel)

Es wurde eine wäßrige Lösung mit 40g Pimelinsäure in 1000ml Wasser vorgelegt und auf 83°C erwärmt bis die Pimelinsäure vollständig gelöst war. Zu dieser Lösung wurde eine wäßrige Nickelhydroxid-Milch (23,2g Ni(OH)$_2$ in 200ml Wasser) unter Rühren hinzugefügt, wodurch Nickel(II)pimelat als weißer Niederschlag ausfiel. Der sedimentierte Niederschlag wurde abgenutscht und bei 130°C im Trockenschrank vorgetrocknet. Abschließend wurde bei 200°C für 24h in einem Vakuumtrockenschrank die Restfeuchte und Kristallwasser entfernt. Auf diese Weise wurde ein grobkörniges getrocknetes Pulver aus Nickel-Pimelat erhalten.

Beispiel 1 b

100 g des getrockneten Nickel-Pimelats aus Beispiel 1 a wurden in 500ml wasserfreiem (Wassergehalt <1Gew.-%) Isopropanol aufgeschlämmt und die Schlämme in eine Kugelmühle gegeben und vermahlen. Dabei entstand eine stabile milchartige Dispersion. REM Aufnahmen zeigen eine Teilchengröße der Teilchen in der Dispersion im Bereich von 75nm. Es wurden in den Proben keine Agglomerate mit einer Teilchengröße von mehr als 0,8μm gefunden.

Beispiel 1 c:

Die milchartige Dispersion nach Beispiel 1b wurde unter Feuchtigkeitsausschluss bei 90°C für 10h im Ablufttrockner getrocknet. Es wurde ein feines weißes Pulver aus Nickel-Pimelat erhalten.

Beispiel 1 d:

Es wurde ein Nickel(II)-Pimelat wie in Beispiel 1c beschrieben hergestellt. Im Unterschied zu Beispiel 1 a wurde der Dispersion von Nickel(II)-Pimelat in Isopropanol 30Gew.-%, bezogen auf das Gewicht des Pimelats, Stearinsäure zugesetzt. Im übrigen wurde in gleicher Weise wie in Beispiel 1 c beschrieben verfahren. Es wurde ein feines weißes Pulver aus Nickel-Pimelat erhalten.

Beispiel 2a (Vergleichsbeispiel)

Das Pulver nach den Beispiel 1a wurde in einem Mischer mit Granulat aus isotaktischem Polypropylenhomopolymer mit einem Schmelzpunkt 162°C und einem MFI von 3g/10min (Exxon Escorene PP 4352 F1) vermischt. Diese Mischung wurde in einem Zweischneckenextruder aufgeschmolzen (Gehäusetemperatur von 240°C und 200 1/min$^{-1}$) und zu stäbchen-förmigen Körnern granuliert. Die Konzentration des Nickel-Pimelats betrug 0,2 Gew.-% bezogen auf das Polypropylen.

REM Aufnahmen an den Granulatkörnern (Probekörper Cast-Folie) zeigen feinverteiltes agglomeratfreies Nickelpimelat in der PP-Matrix. Mittels DSC-Analyse zeigt das Compound aus Polypropylen und Nickelpimelat einen β-Wert von 95.5% beim 2.Aufheizen.

Beispiel 2b

Die Dispersion nach den Beispiel 1 b wurde in einem Mischer auf Granulat aus isotaktischem Polypropylenhomopolymer (Schmelzpunkt 162°C; MFI 3g/10min) aufgetrommelt. Die Konzentration des Nickel-Pimelats betrug 0,2 Gew.-% bezogen auf das Polypropylen. Das Granulat mit dem aufgetrommelten Nickelpimelat wurde wie in Beispiel 2a in einem Zweischneckenextruder aufgeschmolzen (Gehäusetemperatur von 240°C und 200 Umin$^{-1}$) und zu stäbchen-förmigen Körnern granuliert.

REM Aufnahmen an den Granulatkörnern (Probekörper Cast-Folie) zeigen feinverteiltes agglomeratfreies Nickelpimelat in der PP-Matrix. Mittels DSC-Analyse zeigt das Compound aus Polypropylen und Nickelpimelat einen β-Wert von 98.5% beim 2.Aufheizen.

Beispiel 2c

Es wurde Beispiel 2a wiederholt. Anstelle des Nickelpimelat-Pulvers nach Beispiel 1a wurde daß Pulver hergestellt nach Beispiel 1 c verwendet. Im übrigen wurde in gleicher Weise wie in Beispiel 2a beschrieben verfahren.

Auch diese REM Aufnahmen an den Granulatkörnern (Probekörper Cast-Folie) zeigen feinverteiltes agglomeratfreies Nickelpimelat in der PP-Matrix. Mittels DSC-Analyse zeigt das Compound aus Polypropylen und Nickelpimelat einen β-Wert von 98.5% beim 2.Aufheizen.

Beispiel 2d

Es wurde Beispiel 2a wiederholt. Anstelle des Nickelpimelat-Pulvers nach Beispiel 1a wurde daß Pulver hergestellt nach Beispiel 1d verwendet. Zusätzlich wurde die Konzentration des stearinsäurebeschichteten Nickelpimelats auf 0,04 Gew.-% gesenkt. Im übrigen wurde in gleicher Weise wie in Beispiel 2a beschrieben verfahren.

Auch diese REM Aufnahmen an den Granulatkörnern (Probekörper Cast-Folie) zeigen feinverteiltes agglomeratfreies Nickelpimelat in der PP-Matrix. Mittels DSC-Analyse zeigt das Compound aus Polypropylen und Nickelpimelat einen β-Wert von 98.5% beim 2.Aufheizen.

Beispiele 3: Herstellung von Nickeladipat

Beispiel 3a: (Vergleichsbeispiel)

Es wurde eine wäßrige Lösung mit 36,5g Adipinsäure in 1000ml Wasser vorgelegt und auf 83°C erwärmt bis die Adipinsäure vollständig gelöst war. Zu dieser Lösung wurde eine wäßrige Nickelhydroxid-Milch (23,2g Ni(OH)$_2$ in 200ml Wasser) unter Rühren hinzugefügt, wodurch Nickel(II)adipat als weißer Niederschlag ausfiel. Der sedimentierte Niederschlag wurde abgenutscht und bei 130°C im Trockenschrank vorgetrocknet. Abschließend wurde bei 200°C für 24h in einem Vakuumtrockenschrank die Restfeuchte und Kristallwasser entfernt. Auf diese Weise wurde ein grobkörniges getrocknetes Pulver aus Nickel-Adipat erhalten.

Beispiel 3b

100 g des getrockneten Nickel-Adipats aus Beispiel 1 a wurden in 500ml wasserfreiem (Wassergehalt <1Gew.-%) Isopropanol aufgeschlämmt und die Schlämme in eine Kugelmühle gegeben und vermahlen. Dabei entstand eine stabile milchartige Dispersion. REM Aufnahmen zeigen eine Teilchengröße der Teilchen in der Dispersion im Bereich von 60nm. Es wurden in den Proben keine Agglomerate mit einer Teilchengröße von mehr als 0,8μm gefunden.

**Beispiel 3c:**

Die milchartige Dispersion nach Beispiel 1 b wurde unter Feuchtigkeitsausschluss bei 90°C für 10h im Ablufttrockner getrocknet. Es wurde ein feines weißes Pulver aus Nickel-Adipat erhalten.

**Beispiel 3d:**

Es wurde ein Nickel(II)-Adipat wie in Beispielen 1a bis 1 c beschrieben hergestellt. Im Unterschied zu 1b wurde der Dispersion von Nickel(II)-Adipat in Isopropanol 30 Gew.-%, bezogen auf das Gewicht des Adipats, Stearinsäure zugesetzt. Im übrigen wurde in gleicher Weise, wie in den Schritten 1 a bis 1 c beschrieben, verfahren. Es wurde ein feines weißes Pulver aus Nickel-Adipat erhalten.

**Beispiel 4a (Vergleichsbeispiel)**

Das Pulver nach Beispiel 3a wurde in einem Mischer mit Granulat aus isotaktischem Polypropylenhomopolymer mit einem Schmelzpunkt 162°C und einem MFI von 3g/10min (Exxon Escorene PP 4352 F1) vermischt. Diese Mischung wurde in einem Zweischneckenextruder aufgeschmolzen (Gehäusetemperatur von 240°C und 200 1/min$^{-1}$) und zu stäbchen-förmigen Körnern granuliert. Die Konzentration des Nickel-Adipats betrug 0,2 Gew.-% bezogen auf das Polypropylen.

REM Aufnahmen an den Granulatkörnern (Probekörper Cast-Folie) zeigen feinverteiltes agglomeratfreies Nickeladipat in der PP-Matrix. Mittels DSC-Analyse zeigt das Compound aus Polypropylen und Nickeladipat einen β-Wert von 90.0% beim 2.Aufheizen.

**Beispiel 4b**

Die Dispersion nach den Beispiel 3b wurde in einem Mischer auf Granulat aus isotaktischem Polypropylenhomopolymer (Schmelzpunkt 162°C; MFI 3g/10min)aufgetrommelt. Die Konzentration des Nickel-Adipats betrug 0,2 Gew.-% bezogen auf das Polypropylen. Das Granulat mit dem aufgetrommelten Nickeladipat wurde wie in Beispiel 3a in einem Zweischneckenextruder aufgeschmolzen (Gehäusetemperatur von 240°C und 200 1/min$^{-1}$) und zu stäbchen-förmigen Körnern granuliert.

REM Aufnahmen an den Granulatkörnern (Probekörper Cast-Folie) zeigen feinverteiltes agglomeratfreies Nickeladipat in der PP-Matrix. Mittels DSC-Analyse zeigt das Compound aus Polypropylen und Nickeladipat einen β-Wert von 92.5% beim 2.Aufheizen.

**Beispiel 4c**

Es wurde Beispiel 4a wiederholt. Anstelle des Nickeladipat-Pulvers nach Beispiel 3a wurde daß Pulver hergestellt nach Beispiel 3c verwendet. Im übrigen wurde in gleicher Weise wie in Beispiel 4a beschrieben verfahren.

Auch diese REM Aufnahmen an den Granulatkörnern (Probekörper Cast-Folie) zeigen feinverteiltes agglomeratfreies Nickeladipat in der PP-Matrix. Mittels DSC-Analyse zeigt das Compound aus Polypropylen und Nickeladipat einen β-Wert von 92.5% beim 2.Aufheizen.

**Beispiel 4d**

Es wurde Beispiel 4a wiederholt. Anstelle des Nickeladipat-Pulvers nach Beispiel 1 a wurde daß Pulver hergestellt nach Beispiel 3d verwendet. Zusätzlich wurde die Konzentration des stearinsäurebeschichteten Nickeladipats auf 0,04 Gew.-% gesenkt. Im übrigen wurde in gleicher Weise wie in Beispiel 4a beschrieben verfahren.

Auch diese REM Aufnahmen an den Granulatkörnern (Probekörper Cast-Folie) zeigen feinverteiltes agglomeratfreies Nickeladipat in der PP-Matrix. Mittels DSC-Analyse zeigt das Compound aus Polypropylen und Nickeladipat einen β-Wert von 92.5% beim 2.Aufheizen.

Der jeweilige β-Anteil der Compounds nach den Beispielen 1 a bis 1 d sowie 3a bis 3d wurde wie beschrieben über DSC aus der 2. Aufheizkurve bestimmt.

| Beispiel Nr. | Nukleator | Konz. [Gew.:%] | max.β-Anteil (2.Aufheizkurve) [%] |
|---|---|---|---|
| 1 a/2a | Nickel(II)-Pimelat (grobes Pulver) | 0,2 | 95,5 |

(fortgesetzt)

| Beispiel Nr. | Nukleator | Konz. [Gew.: %] | max.β-Anteil (2.Aufheizkurve) [%] |
|---|---|---|---|
| 1 b/2b | Nickel(11)-Pimelat (Dispersion) | 0,2 | 98,5 |
| 1 c/2c | Nickel(II)-Pimelat (feines Pulver) | 0,2 | 98,5 |
| 1 d/2d | Nickel(II)-Pimelat (feines Pulver aus Dispersion mit Stearinsäure) | 0,04 | 98,5 |
| 3a/4a | Nickel(II)-Adipat (grobes Pulver) | 0,2 | 90,0 |
| 3b/4b | Nickel(II)-Adipat (Dispersion) | 0,2 | 92,5 |
| 3c/4c | Nickel(II)-Adipat (feines Pulver) | 0,2 | 92,5 |
| 3d/4d | Nickel(II)-Adipat (feines Pulver aus Dispersion mit Stearinsäure) | 0,04 | 92,5 |

Folienbeispiel 5c

In einem Mischer wurden 20 Gew.-% Compound aus Polypropylen und Nickelpimelat hergestellt nach Beispiel 2a mit 60 Gew.-% Propylenhomopolymer und 20 Gew.-% Propylenblockcopolymer, jeweils bezogen auf die Mischung, vermischt. Diese Mischung wurde in einem Extruder aufgeschmolzen und weiter homogenisiert. Nach dem Extrusionsverfahren wurde die Schmelze aus einer Breitschlitzdüse bei einer Extrusionstemperatur von 245 °C zu einer einschichtigen Folie extrudiert. Diese Folie hatte die folgende Zusammensetzung:

ca. 80 Gew.-%    Propylenhomopolymerisat (PP) mit einem n-heptanlöslichen Anteil von 4,5 Gew.-% (bezogen auf 100 % PP) und einem Schmelzpunkt von 165 °C; und einem Schmelzflußindex von 3,2 g/10 min bei 230 °C und 2,16 kg Belastung (DIN 53 735) und

ca. 20 Gew.-%    Propylen-Ethylen-Blockcopolymerisat mit einem Ethylenanteil von ca. 5 Gew.-% bezogen auf das Blockcopolymer und einem Schmelzflußindex (230°C und 2,16 kg) von 6 g/10min

0,04 Gew.-%    Nickel- Pimelat als β-Nukleierungsmittel

Die Folie enthielt zusätzlich Stabilisator und Neutralisationsmittel in üblichen Mengen.

Die Polymermischung wurde nach der Extrusion über eine erste Abzugswalze und ein weiteres Walzentrio abgezogen, abgekühlt und verfestigt, anschließend längsgestreckt, quergestreckt und fixiert, wobei im einzelnen die folgenden Bedingungen gewählt wurden:

Extrusion:    Extrusionstemperatur 245 °C
Abkühlwalze:    Temperatur 125°C,
Abzugsgeschwindigkeit:    1,5 m/min (Verweilzeit auf der Abzugswalze: 55 sec)
β-Gehalt der Vorfolie:    67%
Längsstreckung:    Streckwalze T = 90 °C
Längsstreckung um den    Faktor 4
Querstreckung:    Aufheizfelder T = 145 °C
Streckfelder    T=145°C
Querstreckung um den    Faktor 4

Die so hergestellte poröse Folie war ca. 30 $\mu$m dick und wies eine Dichte von 0,30 g/cm$^3$ auf und zeigte ein gleichmäßiges weiß-opakes Aussehen. Die Porosität betrug 66% und der Gurley-Wert 200 s. Bei der Folienherstellung traten über mehrere Stunden keine Abrisse auf.

Folienbeispiel 6d

Es wurde eine Folie wie in Folienbeispiel 5c beschrieben hergestellt. Im Unterschied zu Folienbeispiel 5c wurden jetzt das Compound aus Polypropylen und Nickeladipat, hergestellt nach Beispiel 4d, eingesetzt. Die Zusammensetzung und die Verfahrensparameter wurden gegenüber Beispeiel 5c nicht geändert.

Die so hergestellte poröse Folie war ca. 30 $\mu$m dick und wies eine Dichte von 0,32 g/cm$^3$ auf und zeigte ein gleichmäßiges weiß-opakes Aussehen. Die Porosität betrug 63% und der Gurley-Wert 240 s. Bei der Folienherstellung traten über mehrere Stunden keine Abrisse auf.

**Patentansprüche**

1. Verfahren zur Herstellung von Polypropylen mit einem Anteil an $\beta$-kristallinem Polypropylen, bei welchem mindestens ein Me(II)-Dicarbonsäuresalz der Formel $Me^{2+}(^-OOC-(CH_2)_x-COO^-)$, wobei das $Me^{2+}$ ein zweiwertiges Metall einer Nebengruppe ist und x 2 bis 10 beträgt, und mindestens ein Polypropylen gemischt werden und bei einer Temperatur von mindestens 150°C aufgeschmolzen und anschliessend abgekühlt werden, **dadurch gekennzeichnet, daß** die abgekühlte Polypropylenschmelze einen Anteil von mehr als 50 bis <100Gew.-% an $\beta$-kristallinem Polypropylen aufweist, wobei dieser $\beta$-kristalline Polypropylene Anteil mittels DSC-Messung wie in der Beschreibung unter der entsprechenden Überschrift beschrieben bestimmt wird, und das Me(II)-Dicarbonsäuresalz eine mittlere Teilchengröße von 1 bis 500nm aufweist, wobei diese Teilchengröße an einer biaxial verstreckten Folie oder an einer Cast-Folie als Probekörper an einer Rasterelektronischen Mikroskop Aufnahme der Probe bestimmt wird.

2. Verfahren nach Anspruch 1, **dadurch gekennzeichnet dass** beim Abkühlen der Polypropylenschmelze ein $\beta$-Anteil von 70 bis <100 % erzeugt wird.

3. Verfahren nach Anspruch 1 oder 2 **dadurch gekennzeichnet, dass** das Polypropylen eine Mischung aus Propylenhomopolymer und Propylencopolymer und/oder Propylenterpolymer ist.

4. Verfahren nach einem der Ansprüche 1 bis 3, **dadurch gekennzeichnet, dass** das Me(II)-Dicarbonsäuresalz eine mittlere Teilchengröße von 1 bits 30 nm aufweist.

5. Verfahren nach einem der Ansprüche 1 bis 4, **dadurch gekennzeichnet, dass** das Me(II)-Dicarbonsäuresalz ein Adipat oder Pimelat oder Suberat ist.

6. Verfahren zur Herstellung einer verstreckten Flachfolie, bei welchem ein Me(II)-Dicarbonsäuresalz der Formel $Me^{2+}(^-OOC-(CH_2)_x-COO^-)$, wobei das $Me^{2+}$ ein zweiwertiges Metall einer Nebengruppe ist und x 2 bis 10 beträgt, und mindestens ein Polypropylen gemischt werden und in einem Extruder bei einer Temperatur von mindestens 150°C aufgeschmolzen werden und die Schmelze durch eine Flachdüse extrudiert und die Schmelze zu einer Vorfolie mit einem Anteil von mindestens 50% $\beta$-kristallinem Polypropylen abgekühlt wird, wobei dieser $\beta$-kristalline Polypropylene Anteil mittels DSC-Messung wie in der Beschreibung unter der entsprechenden Überschrift beschrieben bestimmt wird, und danach die Vorfolie erwärmt und in Längsrichtung und in Querrichtung verstreckt wird und bei der Verstreckung das $\beta$-kristalline Polypropylen der Vorfolie in die alpha Modifikation des Polypropylens umwandelt, **dadurch gekennzeichnet, daß** das Me(II)-Dicarbonsäuresalz eine mittlere Teilchengröße von 1 bis 500nm aufweist, wobei diese Teilchengröße an der biaxial verstreckten Folie als Probekörper an einer Rasterelektronischen Mikroskop Aufnahme der Folie bestimmt wird.

7. Verfahren nach einem der Ansprüche 1 bis 6, **dadurch gekennzeichnet, dass** die biaxial orientierte Folie porös ist, und/oder wobei der mittlere Porendurchmesser (Bubble Point) im Bereich von 10 bis 350nm liegt.

8. Mischung oder Compound aus Polyolefin und Me(II)-Dicarbonsäuresalz der Formel $Me^{2+}(^-OOC-(CH_2)_x-COO^-)$, wobei das $Me^{2+}$ ein zweiwertiges Metall einer Nebengruppe ist und x 2 bis 10 beträgt, **dadurch gekennzeichnet, daß** das Me(II)-Dicarbonsäuresalz eine mittlere Teilchengröße von 1 bis 500nm aufweist, wobei diese Teilchengröße an einer biaxial verstreckten Folie oder an einer Cast-Folie als Probekörper an einer Rasterelektronischen Mikroskop Aufnahme der Probe bestimmt wird.

9. Mischung oder Compound nach Anspruch 8, **dadurch gekennzeichnet, dass** das zweiwertige Metal Nickel ist.

10. Mischung oder Compound nach Anspruch 8 bis 9, **dadurch gekennzeichnet, dass** das Polyolefin ein isotaktisches Propylenhomopolymer, ein Propylencopolymer, ein Propylenterpolymer oder ein Polyethylen oder eine Mischung aus einem oder mehreren dieser Polyolefine ist.

11. Mischung oder Compound nach einem der Ansprüche 8 bis 10, **dadurch gekennzeichnet, dass** die Mischung oder das Compound einen maximalen $\beta$-Anteil >90% aufweisst, wobei dieser maximale $\beta$-Anteil mittels DSC Messung aus der zweiten Aufheizkurve bestimmt wird.

**12.** Mischung oder Compound nach einem der Anspruch 8 bis 11, **dadurch gekennzeichnet, dass** das Me(II)-Dicarbonsäuresalz eine Me(II)-Pimelat, Me(II)-Adipat oder Me(II)-Suberat ist.

**13.** Mischung oder Compound nach Anspruch 12, **dadurch gekennzeichnet, dass** das Me(II) Nickel ist.

**14.** Verwendung von Me(II)-Dicarbonsäuresalzen der Formel $Me^{2+}(\text{-OOC-(CH}_2)_x\text{-COO}^-)$, wobei das $Me^{2+}$ ein zweiwertiges Metall einer Nebengruppe ist und x 2-bis bis 10 beträgt, als β-Nukleierungsmittel in Polypropylen, **dadurch gekennzeichnet, daß** das Me(II)-Dicarbonsäuresalz eine mittlere Teilchengröße von 1 bis 500nm aufweist, wobei diese Teilchengröße an einer biaxial verstreckten Folie oder an einer Cast-Folie als Probekörper an einer Rasterelektronischen Mikroskop Aufnahme der Proben bestimmt wird.

**15.** Verfahren zur Herstellung von nanoskaligen Me(II)-Dicarbonsäuresalzen der Formel $Me^{2+}(\text{-OOC-(CH}_2)_x\text{-COO}^-)$, wobei das $Me^{2+}$ ein zweiwertiges Metall einer Nebengruppe ist und x 2 bis 10 beträgt, bei dem eine wässrige Lösung einer Dicarbonsäure hergestellt wird und dieser wässrigen Lösung eine Me(II)-Salz-Lösung oder ein Me(II)-Salz hinzugefügt wird und das ausgefallene Me(II)-Dicarbonsäuresalz abgetrennt und getrocknet wird und das getrocknete Me(II)-Dicarbonsäuresalz in einem nicht-wässrigen flüssigen Medium aufgeschlämmt wird und die Schlämme zur Zerkleinerung der aufgeschlämmten Me(II)-Dicarbonsäuresalz bearbeitet wird, wobei das Me(II)-Dicarbonsäuresalz eine mittlere Teilchengröße von 1 bis 500nm aufweist, wobei diese Teilchengröße an einer biaxial verstreckten Folie oder an einer Cast-Folie als Probekörper an einer Rasterelektronischen Mikroskop Aufnahme der Proben bestimmt wird.

**16.** Verfahren nach einem der Ansprüche 1 bis 7 oder 15, dadurch geikennzeichnet, das das zweiwertige Metall Nickel ist.

**Claims**

**1.** A method for manufacturing polypropylene with a content of β-crystalline polypropylene, in which at least one Me (II) dicarboxylic acid salt of formula $Me(II)^{2+}$ ($\text{-OOC-(CH}_2)_x\text{-COO}^-$), wherein the $Me^{2+}$ is a bivalent metal from a subgroup and x has a value from 2 to 10, and at least one polypropylene are mixed, then melted at a temperature of at least 150 °C and then cooled, **characterised in that** the cooled polypropylene melt contains a percentage of more than 50 to < 100 % by weight β-crystalline polypropylene, wherein said percentage of β-crystalline polypropylene is determined by DSC measurement as described in the corresponding section of the description, and the Me(II) dicarboxylic acid salt has an average particle size from 1 to 500 nm, wherein said particle size is determined on a biaxially stretched film or on a cast film as a sample body with an image of the sample taken with a scanning electron microscope.

**2.** The method according to claim 1, **characterised in that** a β-percentage of 70 to < 100 % is produced as the polypropylene melt cools.

**3.** The method according to claim 1 or 2, **characterised in that** the polypropylene is a mixture of a propylene homopolymer and a propylene copolymer and/or a propylene terpolymer.

**4.** The method according to any one of claims 1 to 3, **characterised in that** the Me(II) dicarboxylic acid salt has an average particle size from 1 to 30 nm.

**5.** The method according to any one of claims 1 to 4, **characterised in that** the Me(II) dicarboxylic acid salt is an adipate or a pimelate or a suberate.

**6.** The method for producing a stretched flat film, in which a Me(II) dicarboxylic acid salt having formula $Me(II)^{2+}(\text{-OOC-(CH}_2)_x\text{-COO}^-)$, wherein the $Me^{2+}$ is a bivalent metal from a subgroup and x has a value from 2 to 10, and at least one polypropylene are mixed, then melted in an extruder at a temperature of at least 150 °C, and the melt is extruded through a flat nozzle and the melt is cooled to produce a prefilm with a content of at least 50 % by weight β-crystalline polypropylene, wherein said β-crystalline polypropylene content is determined by DSC measurement as described in the corresponding section of the description, and the prefilm is then heated and stretched lengthwise and transversely, and during the stretching the β-crystalline polypropylene of the prefilm is converted into the alpha modification of the polypropylene, **characterised in that** the Me(II) dicarboxylic acid salt has an average particle size from 1 to 500 nm, wherein said particle size is determined on the biaxially stretched film as a sample body with an image of the film taken with a scanning electron microscope.

7. The method according to any one of claims 1 to 6, **characterised in that** the biaxially oriented film is porous, and/or wherein the average pore diameter (bubble point) is in the range from 10 to 350 nm.

8. A mixture or compound of polyolefin and Me(II) dicarboxylic acid salt having formula $Me(II)^{2+}(^-OOC-(CH_2)_x-COO^-)$, wherein the $Me^{2+}$ is a bivalent metal from a subgroup and x has a value from 2 to 10, **characterised in that** the Me(II) dicarboxylic acid salt has an average particle size from 1 to 500 nm, wherein said particle size is determined on a biaxially stretched film or on a cast film as a sample body with an image of the film taken with a scanning electron microscope.

9. The mixture or compound according to claim 8, **characterised in that** the bivalent metal is nickel.

10. The mixture or compound according to either of claims 8 or 9, **characterised in that** the polyolefin is an isotactic propylene homopolymer, a propylene copolymer, a propylene terpolymer or a polyethylene or a mixture of one or more of said polyolefins.

11. The mixture or compound according to any one of claims 8 to 10, **characterised in that** the mixture or compound has a maximum β-content >90%, wherein said maximum β-content is determined by means DSC measurement from the second heating curve.

12. The mixture or compound according to any one of claims 8 to 11, **characterised in that** the Me(II) dicarboxylic acid salt is an Me(II)-pimelate, an Me(II) adipate, or an Me(II)-suberate.

13. The mixture or compound according to claim 12, **characterised in that** the Me(II) is nickel.

14. A use of Me (II) dicarboxylic acid salts of formula $Me (II)^{2+}(^-OOC-(CH_2)_x-COO^-)$, wherein the $Me^{2+}$ is a bivalent metal from a subgroup and x has a value from 2 to 10, as a β-nucleating agent in polypropylene, **characterised in that** the Me(II) dicarboxylic acid salt has an average particle size from 1 to 500 nm, wherein said particle size is determined on a biaxially stretched film or on a cast film as a sample body with an image of the samples taken with a scanning electron microscope.

15. A method for preparing nanoscale Me(II) dicarboxylic acid salts of formula $Me(II)^{2+}(^-OOC-(CH_2)_x-COO^-)$, wherein the $Me^{2+}$ is a bivalent metal from a subgroup and x has a value from 2 to 10, in which an aqueous solution of a dicarboxylic acid is prepared and an Me (II) salt solution or Me (II) salt is added to said aqueous solution, and the precipitated Me(II) dicarboxylic acid salt is separated and dried, and the dried Me(II) dicarboxylic acid salt is slurried in a nonaqueous liquid medium, and the slurries are processed to crush the slurried Me(II) dicarboxylic acid salt, wherein the Me(II) dicarboxylic acid salt has an average particle size from 1 to 500 nm, wherein said particle size is determined on a biaxially stretched film or on a cast film as a sample body with an image of the samples taken with a scanning electron microscope.

16. The method according to either of claims 1 to 7 or 15, **characterised in that** the bivalent metal is nickel.


**Revendications**

1. Procédé de fabrication d'un polypropylène comportant une proportion de polypropylène bêta-cristallin, ledit procédé consistant à mélanger au moins un sel métallique(II) d'un acide dicarboxylique répondant à la formule $Me^{2+}$ ($^-OOC-(CH_2)_x-COO^-$) , dans laquelle $Me^{2+}$ est un métal de transition divalent et x est compris entre 2 et 10, avec au moins un polypropylène et à les faire fondre à une température d'au moins 150 °C pour ensuite les refroidir, **caractérisé en ce que** dans le polypropylène fondu présente suite à son refroidissement une proportion de polypropylène bêta-cristallin comprise entre 50 et < 100 % en poids, ladite proportion en polypropylène bêta-cristallin étant déterminée en réalisant une mesure par calorimétrie différentielle à balayage telle qu'elle est décrite sous le titre correspondant, et que le sel métallique(II) d'acide dicarboxylique présente une taille des particules moyenne comprise entre 1 et 500 nm, ladite taille des particules étant déterminée en soumettant, en tant qu'éprouvette, un échantillon d'une feuille biaxialement orientée ou d'une feuille non-orientée à une prise de vue par microscopie électronique à balayage.

2. Procédé selon la revendication 1, **caractérisé en ce que** le refroidissement du polypropylène fondu permet d'obtenir une proportion en bêta comprise entre 70 et < 100 %.

**3.** Procédé selon la revendication 1 ou 2, **caractérisé en ce que** ledit polypropylène est un mélange entre un homopolymère de propylène et un copolymère de propylène et/ou un terpolymère de propylène.

**4.** Procédé selon l'une des revendications 1 à 3, **caractérisé en ce que** ledit sel métallique(II) d'acide dicarboxylique présente une taille des particules moyenne comprise entre 1 et 30 nm.

**5.** Procédé selon l'une des revendications 1 à 4, **caractérisé en ce que** ledit sel métallique(II) d'un acide dicarboxylique est un adipate ou pimélate ou subérate.

**6.** Procédé de fabrication d'une feuille plate orientée, consistant à mélanger un sel métallique(II) d'un acide dicarboxylique répondant à la formule $Me^{2+}(^-OOC\text{-}(CH_2)_x\text{-}COO^-)$ , dans laquelle $Me^{2+}$ est un métal de transition divalent et x est compris entre 2 et 10, avec au moins un polypropylène et à les faire fondre dans une extrudeuse à une température d'au moins 150 °C, et à extruder la matière fondue à travers une filière plate puis refroidir la matière fondue pour obtenir une pré-feuille dont la proportion de polypropylène bêta-cristallin est d'au moins 50 %, ladite proportion de polypropylène bêta-cristallin étant déterminée en réalisant une mesure par calorimétrie différentielle à balayage telle qu'elle est décrite sous le titre correspondant, et à réchauffer ensuite ladite pré-feuille pour l'orienter dans le sens longitudinal et dans le sens transversal, ladite orientation de la pré-feuille conduisant à une transformation du bêta-propylène en un propylène ayant subi une modification alpha, **caractérisé en ce que** le sel métallique(II) d'acide dicarboxylique présente une taille des particules moyenne comprise entre 1 et 500 nm, ladite taille des particules étant déterminée en soumettant, en tant qu'éprouvette, ladite feuille biaxialement orientée à une prise de vue par microscopie électronique à balayage.

**7.** Procédé selon l'une des revendications 1 à 6, **caractérisé en ce que** ladite feuille biaxialement orientée est poreuse et/ou le diamètre moyen des pores (point d'ébullition) étant comprise entre 10 et 350 nm.

**8.** Mélange ou formulation complète, constitué(e) d'une polyoléfine et d'un sel métallique(II) d'un acide dicarboxylique répondant à la formule $Me^{2+}(^-OOC\text{-}(CH_2)_x\text{-}COO^-)$, dans laquelle $Me^{2+}$ est un métal de transition divalent et x est compris entre 2 et 10, **caractérisé en ce que** ledit sel métallique(II) d'acide dicarboxylique présente une taille des particules moyenne comprise entre 1 et 500 nm, ladite taille des particules étant déterminée en soumettant, en tant qu'éprouvette, un échantillon d'une feuille biaxialement orientée ou d'une feuille non-orientée à une prise de vue par microscopie électronique à balayage.

**9.** Mélange ou formulation complète selon la revendication 8, caractérisé(e) en ce que ledit métal divalent est le nickel.

**10.** Mélange ou formulation complète selon la revendication 8 à 9, caractérisé(e) en ce que ladite polyoléfine est un homopolymère de propylène isotactique, un copolymère de propylène, un terpolymère de propylène ou un polyéthylène ou un mélange constitué d'une ou de plusieurs desdites polyoléfines.

**11.** Mélange ou formulation complète selon l'une des revendications 8 à 10, caractérisé (e) en ce que ledit mélange ou ladite formulation complète présente une proportion maximale en bêta qui est > 90 %, ladite proportion maximale en bêta étant déterminée à partir de la deuxième courbe de réchauffement d'une mesure par calorimétrie différentielle à balayage.

**12.** Mélange ou formulation complète selon l'une des revendications 8 à 11, caractérisé(e) en ce que ledit sel métallique(II) d'un acide dicarboxylique est un Me (II) -pimélate , Me (II) -adipate ou Me (II) - subérate.

**13.** Mélange ou formulation complète selon la revendication 12, caractérisé(e) en ce que ledit Me(II) est le nickel.

**14.** Utilisation de sels métalliques(II) d'un acide dicarboxylique répondant à la formule $Me^{2+}(^-OOC\text{-}(CH_2)_x\text{-}COO^-)$, dans laquelle $Me^{2+}$ est un métal de transition divalent et x est compris entre 2 et 10, en tant qu'agent de nucléation bêta, **caractérisée en ce que** ledit sel métallique(II) d'acide dicarboxylique présente une taille des particules moyenne comprise entre 1 et 500 nm, ladite taille des particules étant déterminée en soumettant, en tant qu'éprouvette, des échantillons d'une feuille biaxialement orientée ou d'une feuille non-orientée à une prise de vue par microscopie électronique à balayage.

**15.** Procédé de préparation de sels métalliques(II) d'un acide dicarboxylique répondant à la formule $Me^{2+}(^-OOC\text{-}(CH_2)_x\text{-}COO^-)$, dans laquelle $Me^{2+}$ est un métal de transition divalent et x est compris entre 2 et 10, ledit procédé consistant à préparer une solution aqueuse d'un acide dicarboxylique et à ajouter à ladite solution aqueuse une solution d'un sel de Me(II) ou un sel de Me(II) pour ensuite isoler et sécher le sel métallique(II) d'acide dicarboxylique précipité, et à mettre le sel métallique(II) d'acide dicarboxylique, suite à son séchage, en suspension dans un milieu liquide non-aqueux et à soumettre la suspension du sel métallique(II) d'acide carboxylique ainsi obtenue à un traitement visant à réduire la taille des particules en suspension, ledit sel métallique(II) d'acide dicarboxylique présentant une taille des particules moyenne comprise entre 1 et 500 nm, ladite taille des particules étant déterminée en soumettant, en tant qu'éprouvette, des échantillons d'une feuille biaxialement orientée ou d'une feuille non-orientée à une prise de vue par microscopie électronique à balayage.

**16.** Procédé selon l'une des revendications 1 à 7 ou 15, **caractérisé en ce que** ledit métal divalent est le nickel.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 1188278
- US 3540979 A
- DE 3610644
- DE 4420989
- EP 0557721 A

**In der Beschreibung aufgeführte Nicht-Patentliteratur**

- **DOU, Q.** J. Macromolecular Sc. Part B: Physics. Marcel Dekker Inc, 01. November 2007, vol. 46, 1063-1080
- IP.COM Journal. IP.COM INC, 02. September 2004
- **DOU, Q.** J. Elastomers and Plastics. SAGE Publications Ltd. GB, 13. Juli 2009, vol. 41
- *Macromol. Mater. Eng.,* 2000, vol. 275, 8-17
- *GAK 5/1988 Jahrgang 41,* 211 ff
- *Macromol. Rapid Commun,* 2001, vol. 22, 176-180